(19) Europäisches Patentamt
European Patent Office
Office européen des brevets

(11) EP 0 798 041 A1

(12) **EUROPÄISCHE PATENTANMELDUNG**

(43) Veröffentlichungstag:
**01.10.1997 Patentblatt 1997/40**

(51) Int. Cl.[6]: **B01J 31/22**, B01J 31/18, C07C 67/343

(21) Anmeldenummer: **97200759.5**

(22) Anmeldetag: **13.03.1997**

(84) Benannte Vertragsstaaten:
**DE FR GB**

(30) Priorität: **25.03.1996 DE 19611629**

(71) Anmelder: **HOECHST AKTIENGESELLSCHAFT**
**65929 Frankfurt am Main (DE)**

(72) Erfinder:
- **Seitz, Thomas, Dr.**
  **68542 Heddesheim (DE)**
- **Bogdanovic, Sandra, Dr.**
  **60322 Frankfurt (DE)**
- **Beller, Matthias, Prof. Dr.**
  **85748 Garching (DE)**
- **Kleiner, Hans-Jerg, Dr.**
  **61476 Kronberg (DE)**

(54) **Heterocyclische Carbene enthaltende Metallkomplexe als Katalysatoren für C-C-Verknüpfungen**

(57) Ein Katalysator für Kohlenstoff-Kohlenstoff-Verknüpfungsreaktionen, enthaltend eine Komplexverbindung der Formel (I)

$$[L_aM_bX_c]^{n+}(A)_n \qquad (I)$$

worin die Symbole und Indizes folgende Bedeutungen haben:

M ist gleich oder verschieden Pd, Rh, Ni, Ru, Co;
X bedeutet gleich oder verschieden an das Zentralatom gebundene ein- oder mehrzähnige, geladene oder ungeladene Liganden;
A ist ein einfach geladenes Anion oder das chemische Äquivalent eines mehrfach geladenen Anions,
b ist eine natürliche Zahl von 1 bis 3;
a ist eine natürliche Zahl von 1 bis 5 • b;
c ist Null oder eine natürliche Zahl von 1 bis 4 • b;
n ist Null oder eine natürliche Zahl von 1 bis 6, mit der Maßgabe, daß $c + n > 0$ ist, und
L ist gleich oder verschieden ein cyclisches Monocarben mit 5, 6 oder 7 Ringgliedern mit mindestens einem Stickstoffatom in Nachbarschaft zu dem Carbenkohlenstoff oder ein Dicarben, bei dem zwei solcher cyclischen Monocarbene über eine Brücke verbunden sind, mit Ausnahme solcher Verbindungen, in denen das cyclische Carben ein Imidazol- oder Pyrazolsystem enthält.



Printed by Rank Xerox (UK) Business Services
2.14.15/3.4

## Beschreibung

Komplexverbindungen aus einem Übergangsmetall als Zentralatom und an dieses gebundene Steuerliganden haben in jüngster Zeit als Homogenkatalysatoren vielfache Anwendung gefunden. Ihre Bedeutung liegt insbesondere bei Reaktionen, die zum Aufbau von CC-, CH-, NC- und OC-Bindungen führen. Beispiele für technische Prozesse, die in Gegenwart derartiger Katalysatoren durchgeführt werden, sind die Hydrierung und die Hydroformylierung von ungesättigten organischen Verbindungen, vorzugsweise von Olefinen. Als Steuerliganden, die, zumeist im Überschuß angewandt, gleichzeitig auch die Komplexverbindung stabilisieren und neben dem Zentralatom maßgebend für die spezifische katalytische Wirksamkeit sind, finden bisher nahezu ausschließlich organische Amine, Phosphane oder Phosphite Anwendung. Bekannte Beispiele sind Komplexverbindungen der allgemeinen Formel $ClRhL_3$, die als Hydrierkatalysatoren, und $H(CO)RhL_3$, die als Hydroformylierungskatalysatoren wirken, wobei L in beiden Fällen für ein Triarylphosphan steht.

Organische Phosphane haben sich als Steuerliganden wegen ihrer stofflichen Vielfalt, ihrer katalytischen Wirksamkeit und ihrer Selektivität in der industriellen Praxis gut bewährt. Dennoch stehen ihrer verbreiteten Anwendung eine Reihe Nachteile entgegen. Unter ihnen ist insbesondere die Oxidationsempfindlichkeit zu nennen, die vor allem in Gegenwart von Metallen und Metall-Ionen auftritt. Daher müssen bei Verwendung von Katalysatoren auf Basis Phosphane enthaltender Komplexverbindungen Maßnahmen zum Ausschluß von Oxidationsmitteln, wie Sauerstoff oder Luft, ergriffen werden, um die Verluste der häufig nur kostenaufwendig herzustellenden Liganden zu verringern. Eine weitere Eigenschaft, die allen organischen Phosphanen eigen ist und ihre Einsatzmöglichkeit begrenzt, ist die irreversible Spaltung von Phosphor-Kohlenstoff-Bindungen, die z.B. bei der Hydroformylierung oberhalb bestimmter, von der Art des Phosphans abhängiger Temperaturen verstärkt auftritt und zur Deaktivierung des Katalysators und damit die Wirtschaftlichkeit des Verfahrens beeinträchtigendem hohen Phosphanverbrauch führt. Schließlich erlauben die herkömmlichen Alkyl- und Arylphosphane ebensowenig wie die gleichfalls als Liganden eingesetzten organischen Phosphite der allgemeinen Formel $P(OR)_3$ (wobei R für Alkyl- oder Arylreste steht) den gesamten Bereich der elektronischen Steuerungsmöglichkeiten hinsichtlich der katalytisch aktiven Metallzentren abzudecken.

Es bestand daher die Aufgabe, neue Katalysatorsysteme zu entwickeln, die die geschilderten Nachteile nicht aufweisen und überdies synthetisch leicht und kostengünstig zugänglich sind. Zudem soll die Konstitution der Steuerliganden auf einfache Weise verändert werden können, so daß es möglich ist, Metallkomplexe herzustellen, die individuelle katalytische Probleme lösen.

Es wurde nun überraschend gefunden, daß sich bestimmte, cyclische Mono- oder Dicarbene enthaltende Metallkomplexe in besonderer Weise zur Katalyse von Kohlenstoff-Kohlenstoff-Verknüpfungsreaktionen eignen.

Katalytisch wirksame Metallkomplexe mit Carbenliganden, die sich von Imidazol und Pyrazol ableiten, werden in den nicht vorveröffentlichten deutschen Patentanmeldungen P 44 47 066.5 und P 44 47 070.3 vorgeschlagen.

Gegenstand der Erfindung ist ein Katalysator für Kohlenstoff-Kohlenstoff-Verknüpfungsreaktionen, enthaltend eine Komplexverbindung der Formel (I),

$$[L_aM_bX_c]^{n+}(A)_n \qquad (I)$$

worin die Symbole und Indizes folgende Bedeutungen haben:

- M ist gleich oder verschieden Pd, Rh, Ni, Ru, Co;
- X bedeutet gleich oder verschieden an das Zentralatom gebundene ein- oder mehrzähnige, geladene oder ungeladene Liganden;
- A ist ein einfach geladenes Anion oder das chemische Äquivalent eines mehrfach geladenen Anions;
- b ist eine natürliche Zahl von 1 bis 3;
- a ist eine natürliche Zahl von 1 bis $5 \cdot b$;
- c ist Null oder eine natürliche Zahl von 1 bis $4 \cdot b$;
- n ist Null oder eine natürliche Zahl von 1 bis 6, mit der Maßgabe, daß $c + n > 0$ ist, und
- L ist gleich oder verschieden ein cyclisches Monocarben mit 5, 6 oder 7 Ringgliedern mit mindestens einem Stickstoffatom in Nachbarschaft zu dem Carbenkohlenstoff oder ein Dicarben, bei dem zwei solcher cyclischen Monocarbene über eine Brücke verbunden sind, mit Ausnahme solcher Verbindungen, in denen das cyclische Carben ein Imidazol- oder Pyrazolsystem ist.

Die neuen Verbindungen sind in organischen Lösungsmitteln, aber auch in Wasser löslich, insbesondere wenn sie durch Sulfonatreste substituierte, aliphatische oder aromatische Reste enthalten. Sie zeichnen sich durch erhebliche Thermostabilität, teilweise bis oberhalb 350°C, hohe Oxidationsstabilität und ausgeprägte katalytische Aktivität bei Reaktionen aus, die zum Aufbau von Kohlenstoff-Kohlenstoff-Bindungen führen. Außerdem neigen die neuen Verbindungen im Gegensatz zu Phosphan- und Phosphit-Komplexverbindungen nicht zur Dissoziation, so daß ein Ligandenüberschuß zur Steuerung der Reaktivität und zur Stabilisierung nicht erforderlich ist. Dieses Verhalten der

beanspruchten Carben-Metallkomplexe war nicht vorauszusehen, denn Komplexverbindungen, die Carben-Liganden enthalten, sind als Katalysatoren für die Olefin- und Alkin-Metathese bekannt, also eine Reaktion, bei der Kohlenstoff-Kohlenstoff-Mehrfachbindungen enthaltende Moleküle gespalten werden, und zwar unter entscheidender Beteiligung des Carben-Liganden (siehe z.B. J. Am. Chem. Soc. 1991, 113, 6899 und J. Am. Chem. Soc. 1993, 115, 9858).

Der das Carben-Kohlenstoffatom enthaltende Ring ist gesättigt, ungesättigt oder aromatisch, unsubstituiert oder substituiert und ist gegebenenfalls auch Teil eines anellierten Ringsystems.

Bevorzugte Substituenten sind gleiche oder verschiedene, geradkettige oder verzweigte, gegebenenfalls sulfonierte Alkylreste mit vorzugsweise 1 bis 7 Kohlenstoffatomen, gegebenenfalls sulfonierte aliphatische mono- oder polycyclische Reste mit vorzugsweise 5 bis 18 Kohlenstoffatomen, gegebenenfalls sulfonierte Alkenylgruppen mit vorzugsweise 2 bis 5 Kohlenstoffatomen, gegebenenfalls sulfonierte Arylgruppen mit vorzugsweise 6 bis 14 Kohlenstoffatomen oder gegebenenfalls sulfonierte Arylalkylreste mit vorzugsweise 7 bis 19 Kohlenstoffatomen.

Jeweils zwei, vorzugsweise benachbarte Substituenten zusammen können ebenfalls gemeinsam gleiche oder verschiedene anellierte und gegebenenfalls sulfonierte Reste mit vorzugsweise 3 bis 7 Kohlenstoffatomen bedeuten.

Besonders bevorzugt sind die folgenden 5-, 6- und 7-gliedrigen Ringsysteme, die jeweils wie oben angegeben substituiert sein können:

a) Fünfringsysteme aus der Gruppe:

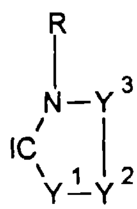 , 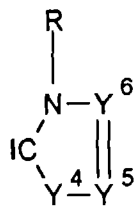 , 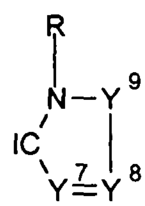

wobei

$Y^1$, $Y^2$, $Y^3$, $Y^4$, $Y^9$ -NR-, -S-, $-CH_2-$, -CHR'-, -CR'R'- und
$Y^5$, $Y^6$, $Y^7$, $Y^8$ gleich oder verschieden = N-, -CH =, = CR' bedeuten;

b) Sechsringsysteme aus der Gruppe:

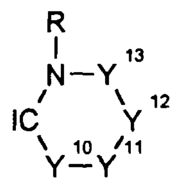 , 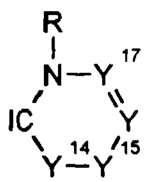 , 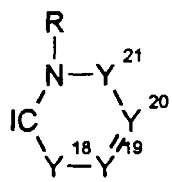 , 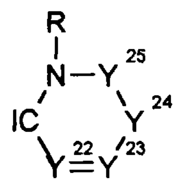 , 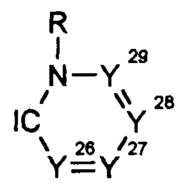

wobei

$Y^{10, 11, 12, 13, 14, 15, 18, 21, 24, 25}$ gleich oder verschieden -NR-, -S-, $-CH_2-$, -CHR'-, -CR'R'- und
$Y^{16, 17, 19, 20, 22, 23, 26, 27, 28, 29}$ gleich oder verschieden = N-, = CH-, = CR'- bedeuten;

c) Siebenringsysteme aus der Gruppe:

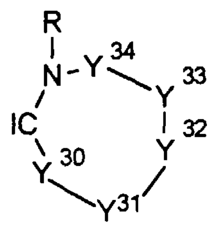 , 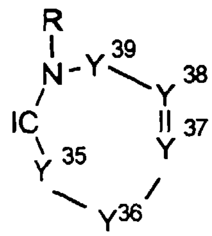 , 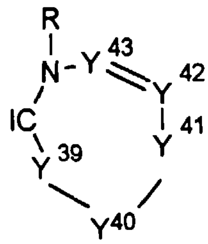 , 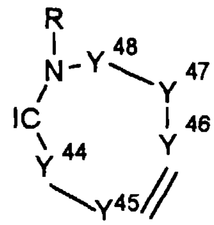 ,

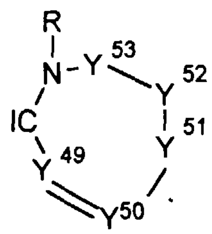 , 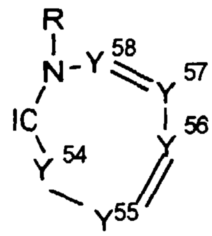 , 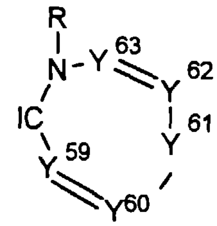 , 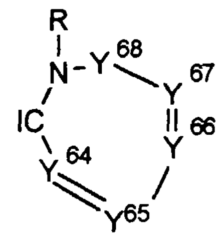

wobei

| | |
|---|---|
| $Y^{30-34, 35, 36, 38-41, 44, 47, 48, 51-54, 61, 68}$ | gleich oder verschieden -NR-, -S-, $-CH_2-$, -CHR'-, -CR'R'-, |
| $Y^{32, 33, 41, 47, 51, 52, 61}$ | auch -O- und |
| $Y^{37, 38, 42, 43, 45, 46, 49, 50, 55-60, 62-67}$ | gleich oder verschieden = N-, -CH =, -CR' bedeuten, mit den Maßgaben, daß Schwefel- und/oder Sauerstoffatome nicht benachbart sein dürfen und daß ein Ring nicht mehr als vier Stickstoffatome enthalten darf; |
| R,R' | sind gleich oder verschieden H, geradkettige oder verzweigte, gegebenenfalls sulfonierte Alkylreste mit vorzugsweise 1 bis 7 Kohlenstoffatomen, gegebenenfalls sulfonierte aliphatische mono- oder polycyclische Reste mit vorzugsweise 5 bis 18 Kohlenstoffatomen, gegebenenfalls sulfonierte Alkenylgruppen mit vorzugsweise 2 bis 5 Kohlenstoffatomen, gegebenenfalls sulfonierte Arylgruppen mit vorzugsweise 6 bis 14 Kohlenstoffatomen oder gegebenenfalls sulfonierte Arylalkylreste mit vorzugsweise 7 bis 19 Kohlenstoffatomen bedeuten, |
| R' | kann auch F, Cl oder eine geradkettige oder verzweigte Alkoxygruppe mit 1 bis 7 Kohlenstoffatomen sein, zwei benachbarte Reste R und/oder R' können gemeinsam jeweils auch gleiche oder verschiedene anellierte und gegebenenfalls sulfonierte Reste mit 3 bis 7 Kohlenstoffatomen bedeuten. |

Ganz besonders bevorzugt sind die folgenden Systeme:

a) 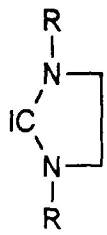 , 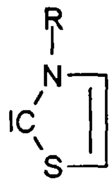 , 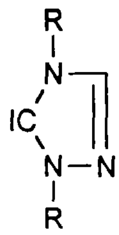 , 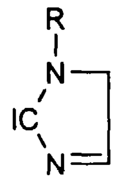 ,

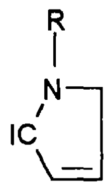 , 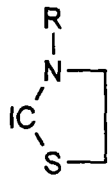 , 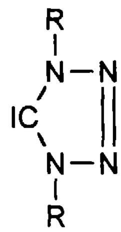 , 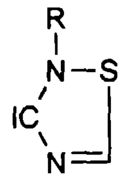

b) 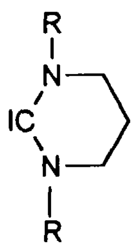 , 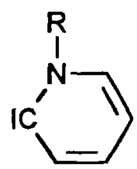 , 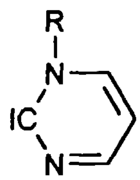 , 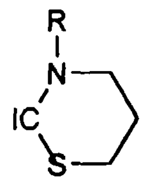 , 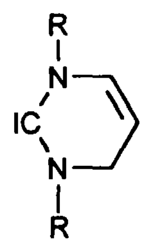

c) 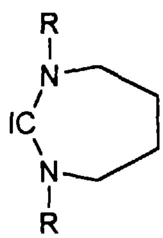 , 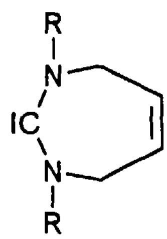 , 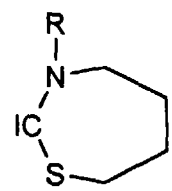 , 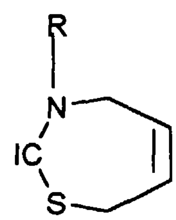 .

Insbesondere bevorzugt ist

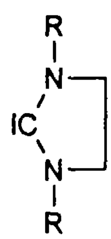 ,

wobei ein bis vier Wasserstoffatome des Ringsystems gegebenenfalls durch gleiche oder verschiedene Reste R' ersetzt sein können und wobei R und R' die oben angegebenen Bedeutungen haben.

Im Fall von Dicarbenen ist die Brücke zwischen zwei Monocarbenen vorzugsweise eine Gruppe Y

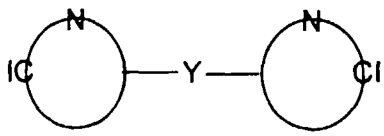

wobei Y ein gesättigter oder ungesättigter, geradkettiger oder verzweigter, offenkettiger oder cyclischer Alkylen- oder Alkylidenrest mit vorzugsweise 1 bis 8, besonders bevorzugt 1 bis 4, Kohlenstoffatomen ist, wobei eine oder zwei - $CH_2$-Gruppen durch -O-, -S-, $SiR''_2$ (mit R'' gleich oder verschieden $C_{1-12}$-Alkyl, Cycloalkyl, Aryl oder Aralkyl) und ein oder mehrere H-Atome durch F ersetzt sein können oder eine gegebenenfalls substituierte Arylengruppe mit 4 bis 20 Kohlenstoffatomen, die auch ein oder mehrere Heteroatome, vorzugsweise N, enthalten kann, vorzugsweise eine Phenylengruppe.

Ein- oder mehrzähnige Liganden, die neben den Carbenen in den Komplexverbindungen enthalten sein können und in der allgemeinen Formel (I) durch X wiedergegeben werden, sind vorzugsweise Wasserstoff oder das Wasserstoff-Ion, Halogene oder Halogen-Ionen, Pseudohalogenide, Carboxylat-Ionen, Sulfonat-Ionen, Amidreste, Alkoholatreste, Acetylacetonatrest, Kohlenmonoxid, Alkylreste mit 1 bis 7 Kohlenstoffatomen, Stickstoffmonoxid, Nitrile, Isonitrile, Mono- oder Diolefine, Alkine und $\pi$-Aromatenreste. Sind mehrere dieser Liganden im Komplexmolekül enthalten, dann können sie gleich oder verschieden sein.

Die Reste R und R' stehen vorzugsweise für H, Methyl, Isopropyl, tert.-Butyl, Benzyl, Triphenylmethyl, Phenyl, Tolyl, Xylyl, Mesityl und Adamantyl.

Jeweils zwei Reste R und/oder R' können zusammen mit zwei benachbarten Atomen des cyclischen Carbens ein Ringsystem bilden. Sind die beiden Atome im Ring durch eine Doppelbindung verknüpft, stehen die Reste vorzugsweise für $-(CH)_4-$, was zur Ausbildung eines aromatischen Sechsrings führt; sind die beiden Atome im Carbenring durch eine Einfachbindung verbunden, stehen die Reste vorzugsweise für $-(CH_2)_4-$ und $-(CH_2)_5-$.

Die durch Y bezeichneten Brückenglieder der Dicarbene sind vorzugsweise die Methylen-, Ethylen-, Diphenylmethylen-, 1,3-Phenylen- und die Ethylidengruppe. Unter den Silizium enthaltenden Brückengliedern werden die Dimethylsilylen- und die Tetramethyldisilylengruppe bevorzugt.

a ist vorzugsweise 1 oder 2, b vorzugsweise 1, n steht insbesondere für die Zahlen 0 bis 3.

A vertritt bevorzugt Halogenid-, Pseudohalogenid-, Tetraphenylborat-, Tetrafluoroborat-, Hexafluorophosphat- und Carboxylat-Ionen, unter den zuletzt genannten insbesondere das Acetat-Ion, ferner Metallkomplex-Anionen, wie Tetracarbonyl-Cobaltat, Hexafluoroferrat(III), Tetrachloroferrat(III), Tetrachloroaluminat oder Tetrachloropalladat(II).

Die erfindungsgemäß eingesetzten Verbindungen können nach an sich bekannten, dem Fachmann geläufigen Methoden hergestellt werden. Dabei kann auch von hier nicht aufgeführten, dem Fachmann bekannten Varianten Gebrauch gemacht werden. Sie sind auf verschiedenen Wegen zugänglich. Eine Herstellungsvariante geht von einfachen Verbindungen, d.h. Salzen oder Metallkomplexen (wie den Acetylacetonaten, Metallcarbonylen) jenes Elements aus, das das Zentralatom oder die Komplexverbindung bildet. Nach einer anderen Variante erhält man die neuen Verbindungen aus Komplexverbindungen durch Ligandenaustausch oder durch Eliminierungs- und/oder Substitutionsreaktionen, zum Beispiel aus gängigen Solvenskomplexen dieser Metallverbindungen wie $PdCl_2\ (C_6H_5C = N)_2$, $NiBr_2 \cdot 2\ DMF$ (DMF = Dimethylformamid) oder $Cl_2Pt[(CH_3)_2NCH_2CH_2N(CH_3)_2]$. Ferner entstehen solche Verbindungen durch einfache Addition eines Carbens an die jeweilige Metallkomponente, wobei diese Anlagerung auch unter Aufbrechen einer Brückenstruktur erfolgen kann.

Die Carbene werden entsprechend ihrer Beständigkeit entweder in freier Form als Lösung eingesetzt oder, häufiger, im Reaktionsgemisch aus Verbindungen hergestellt, die unter den Reaktionsbedingungen in Carbene überführt werden können.

Hierzu eignet sich beispielsweise folgende allgemein anwendbare Synthesesequenz:

X = N, S

Y = OH, OR, $NR_2$, $CCl_3$, CN

(siehe auch Houben-Weyl, "Methoden der Organischen Chemie", E19b, S. 1773 ff. / Thieme Verlag, Stuttgart 1989).

Zweistufige $\alpha$-Eliminierung aus cyclischen Amminiumsalzen führt zu den freien, heteroatomstabilisierten Carbenen, die gegebenenfalls auch als Dimere vorliegen.

Diese sind im Fall von ausschließlich Stickstoff enthaltenden Systemen aus den entsprechenden Diaminen durch Kondensationsreaktion zugänglich

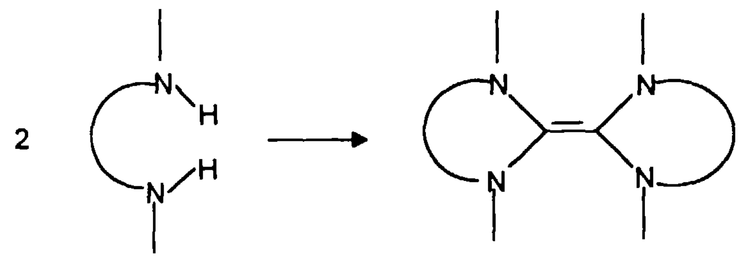

wobei als Kondensationsmittel beispielsweise Orthoameisensäuretriethylester (siehe z.B. Chem. Ber. 1963, 96(5), 1208; DE-A 11 63 331) oder N,N-Dimethylformamide-dimethylacetal (siehe z.B. US-A 3,239,519) geeignet sind.

Die Umsetzung der Ausgangsstoffe, d.h. der einfachen Salze bzw. der Komplexverbindungen, mit den Carbenen bzw. deren Vorstufen und gegebenenfalls weiteren Liganden erfolgt durch Mischen der Reaktanten in einem Lösungsmittel bei Raumtemperatur oder erhöhter Temperatur. Die Reaktion verläuft im allgemeinen mit hoher Geschwindigkeit und ist im wesentlichen oft nach wenigen Minuten beendet. Es empfiehlt sich jedoch zur Vervollständigung der Reaktion Umsetzungszeiten von bis zu mehreren Stunden einzuhalten, insbesondere wenn die Einsatzstoffe im verwendeten Medium nur teilweise gelöst sind, d.h. aus Suspension reagieren.

Zur Herstellung sulfonierter Liganden enthaltender Komplexverbindungen, die wasserlöslich sind, setzt man als Ausgangsstoffe zumindest einen Reaktionspartner ein, dessen Molekül oder Molekülfragment sulfoniert ist.

Zur Isolierung der Komplexverbindungen aus dem Reaktionsmedium hat es sich bewährt, das Lösungsmittel im Hochvakuum zu entfernen. Das Rohprodukt wird zur Reinigung gewaschen und aus einem geeigneten Lösungsmittel oder Lösungsmittelgemisch, das im Einzelfall durch routinemäßige Vorversuche zu ermitteln ist, umkristallisiert.

Die Synthese der Metallcarbenkomplexe kann beispielsweise aus den entsprechenden Alkenvorstufen erfolgen (siehe z.B. J. Chem. Soc. Dalton Trans. 1974, 1827):

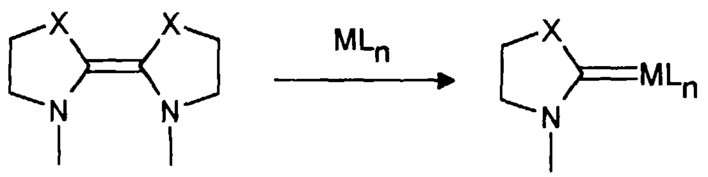


(X = S oder N-R, wobei M, L, R und n die oben angegebenen Bedeutungen haben)

Beispiele für Ausgangsverbindungen sind:

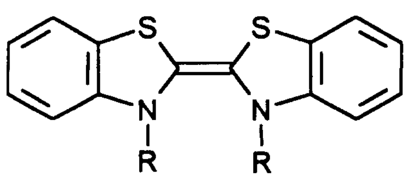

**Liebigs Ann. Chem. 1967, 708, 155**

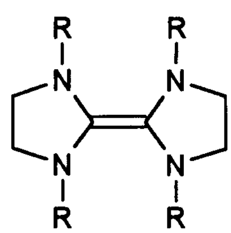

**für R = Me: US-A 3,239,519**

**für R = Ph: DE -A 22 63 331**

Sechsring-Carbene sind beispielsweise aus Chlorpyrimidin erhältlich (siehe z.B. J. Organomet. Chem. 1985, 291, 259):

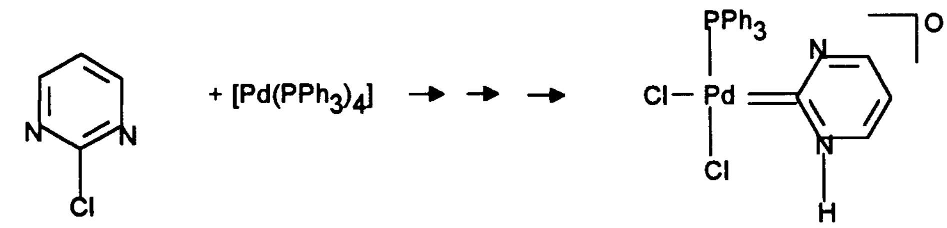

In Anlehnung an Liebigs Ann. Chem. 1967, 708, 155 und J. Chem. Soc. Dalton Trans. 1974, 1827 können Dicarbenkomplexe beispielsweise nach folgendem Schema hergestellt werden.

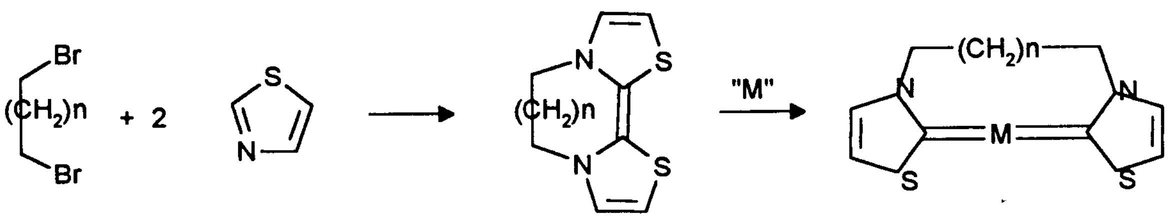


Die oben beschriebenen Katalysatoren finden Verwendung in einem Verfahren zur Knüpfung von Kohlenstoff-Kohlenstoff-Bindungen, beispielsweise durch Hydroformylierung von Olefinen oder die Heck-Reaktion.

Ein solches Verfahren ist ebenfalls Gegenstand der Erfindung.

Bevorzugt ist eine Variante dieses Verfahrens, bei der die Katalysatorsysteme der Formel (I) bei der Reaktion in situ aus entsprechenden Metallverbindungen und einer Carbenvorstufe gebildet werden.

Als Carbenvorstufe eignen sich Alkene der allgemeinen Formel (II)

$$Z = Z \qquad \text{(II)}$$

Die Gruppe Z ist dabei ein Ringsystem mit 5, 6 oder 7 Ringgliedern, wobei das Kohlenstoffatom, an dem sich die exocyclische Doppelbindung befindet, direkt an ein Stickstoff- und ein weiteres Heteroatom gebunden ist und wobei die beiden Gruppen Z auch über eine Brücke verbunden sein können; ausgenommen sind dabei Verbindungen in denen Z ein Imidazol- oder Pyrazolsystem ist.

Bevorzugt enthält die Gruppe Z keine Heteroatome außer den beiden, die an das Kohlenstoffatom gebunden sind, welches die exocyclische Doppelbindung trägt. Heteroatome sind bevorzugt N und S.

Bevorzugt ist Z eines der folgenden 5-, 6- oder 7-gliedrigen Ringsysteme:

a) Fünfringsysteme aus der Gruppe:

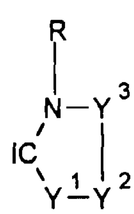 , 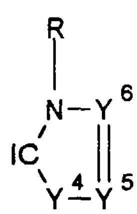 , 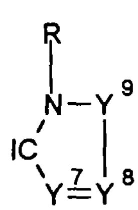

wobei

| | |
|---|---|
| $Y^1$ | gleich oder verschieden -NR-, -S-; |
| $Y^4$ | -S-; |
| $Y^7$ | =N-; |
| $Y^2$, $Y^3$, $Y^9$ | gleich oder verschieden $-CH_2-$, -CHR'-, -CR'R'- und |

$Y^5$, $Y^6$, $Y^8$ gleich oder verschieden -CH =, = CR' bedeuten;

b) Sechsringsysteme aus der Gruppe:

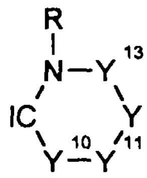 , 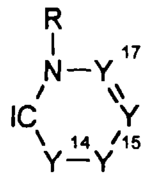 , 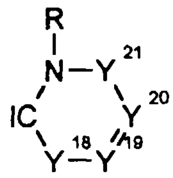 , 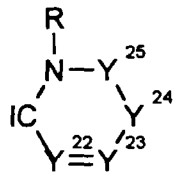 , 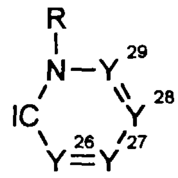

wobei

| | |
|---|---|
| $Y^{10, 14, 18}$ | gleich oder verschieden -NR-, -S-; |
| $Y^{22, 26}$ | = N-; |
| $Y^{11, 12, 13, 15, 21, 24, 25}$ | gleich oder verschieden $-CH_2-$, -CHR'-, -CR'R'- und |
| $Y^{16, 17, 19, 20, 23, 27, 28, 29}$ | gleich oder verschieden = CH-, = CR'-bedeuten; |

c) Siebenringsysteme aus der Gruppe:

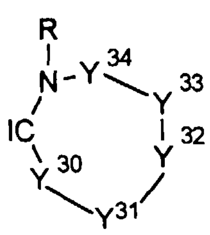 , 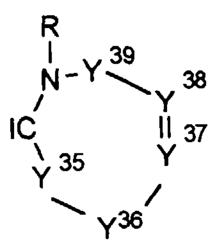 , 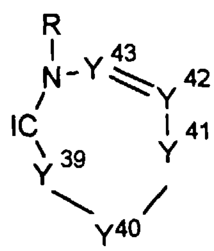 , 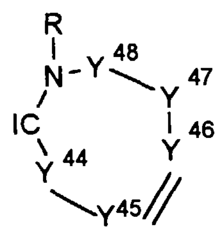 ,

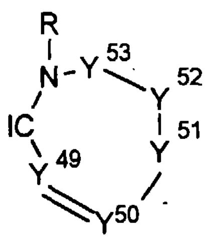 , 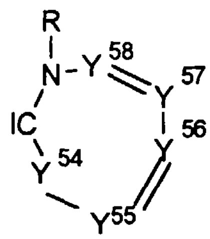 , 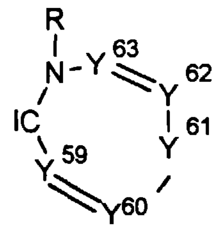 , 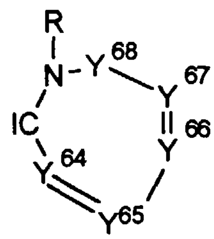

wobei

| | |
|---|---|
| $Y^{30, 35, 39, 44, 54}$ | gleich oder verschieden -NR-, -S-; |
| $Y^{49, 59, 64}$ | =N-; |
| $Y^{31-34, 36, 38, 40, 41, 47, 48, 51-53, 61, 68}$ | gleich oder verschieden $-CH_2-$, -CHR'-, -CR'R'- ; |
| $Y^{37, 38, 42, 43, 45, 46, 50, 55-60, 62-67}$ | gleich oder verschieden -CH =, -CR' = bedeuten, |
| R,R' | sind gleich oder verschieden H, geradkettige oder verzweigte, gegebenenfalls sulfonierte Alkylreste mit vorzugsweise 1 bis 7 Kohlenstoffatomen, gegebenenfalls sulfonierte aliphatische mono- oder polycyclische Reste mit vorzugsweise 5 bis 18 Kohlenstoffatomen, gegebenenfalls sulfonierte Alkenylgruppen mit vorzugsweise 2 bis 5 Kohlenstoffatomen, gegebenenfalls sulfonierte Arylgruppen mit vorzugsweise 6 bis 14 Kohlenstoffatomen oder gegebenenfalls sulfonierte Arylalkylreste mit vorzugsweise 7 bis 19 Kohlenstoffatomen bedeuten, |

R' kann auch F, Cl oder eine geradkettige oder verzweigte Alkoxygruppe mit 1 bis 7 Kohlenstoffatomen sein, zwei benachbarte Reste R und/oder R' können gemeinsam jeweils auch gleiche oder verschiedene anellierte und gegebenenfalls sulfonierte Reste mit 3 bis 7 Kohlenstoffatomen bedeuten.

Ganz besonders bevorzugt sind die folgenden Systeme, die gegebenenfalls mit einem oder mehreren Resten R,R' substituiert sein können:

a) 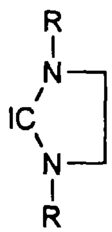 , 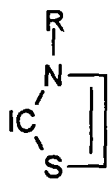 , 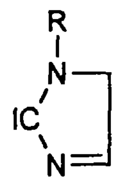 , 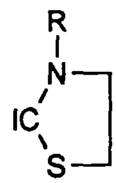 ,

b) 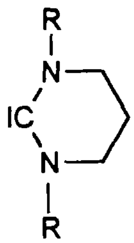 , 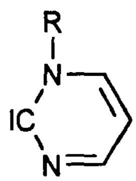 , 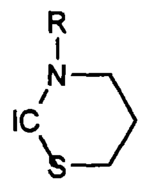 , 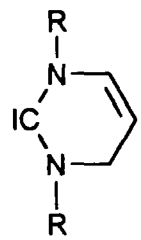 ,

c) 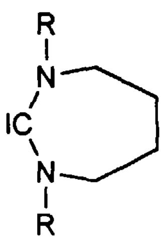 , 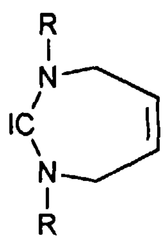 , 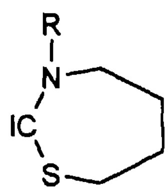 , 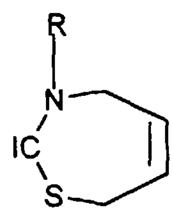 .

Insbesondere bevorzugt ist

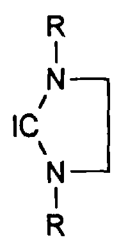 ,

wobei ein bis vier Wasserstoffatome des Ringsystems gegebenenfalls durch gleiche oder verschiedene Reste R' ersetzt sein können, wobei R und R' die oben angegebenen Bedeutungen haben.

Weitere Bevorzugungen, beispielsweise für eine etwaige Brücke, ergeben sich aus den bei den Verbindungen der Formel (I) genannten.

Durch das erfindungsgemäße Verfahren entfallen die aufwendige Katalysator- bzw. Carbenpräformierung, zudem liegt das Carben in einer sehr stabilen Lagerform vor.

a) Verwendung von Verbindungen der Formel (I) bei der Hydroformylierung

Es ist bekannt, durch Umsetzung von Olefinen mit Kohlenmonoxid und Wasserstoff (Hydroformylierung) Aldehyde und Alkohole herzustellen, die ein Kohlenstoffatom mehr als das Ausgangsolefin enthalten. Die Reaktion wird durch Hydridometallcarbonyle, vorzugsweise der Metalle der Gruppen 8, 9, 10 des Periodensystems (entsprechend der IUPAC-Empfehlung von 1985) katalysiert. Neben Kobalt, das als Katalysatormetall ursprünglich und in großem Umfang technisch Verwendung fand, gewinnt in neuerer Zeit Rhodium zunehmend Bedeutung.

Die Kobalt- oder Rhodium-Komplexverbindungen der Formel (I) werden vorzugsweise nach dem oben angegebenen Verfahren in situ aus einem entsprechenden Alken und Metallsalz im Reaktionsgemisch der Hydroformulierung hergestellt. Sie können jedoch auch vor der eigentlichen Reaktion synthetisiert werden.Die Herstellung der Katalysatoren erfolgt in beiden Fällen wie oben beschrieben aus Salzen oder Komplexverbindungen der Metalle Kobalt bzw. Rhodium. Unter der Einwirkung von Synthesegas gehen die ursprünglich eingesetzten Kobalt- oder Rhodium-Komplexverbindungen in den aktiven Hydroformylierungskatalysator über.

Die Konzentration des Metalls (in Form der Komplexverbindung) in der organischen oder wäßrigen Katalysatorlösung beträgt, bezogen auf eingesetzte olefinisch ungesättigte Verbindung, $10^{-6}$ bis 1 mol-%, vorzugsweise $10^{-4}$ bis $10^{-1}$ mol-%. Die erforderliche Kobaltkonzentration ist innerhalb der angegebenen Bereiche im allgemeinen bis zu einer Zehnerpotenz höher als die erforderliche Rhodiumkonzentration.

Zur Durchführung des erfindungsgemäßen Verfahrens werden im allgemeinen Alken und Metallsalze in Lösung vorgelegt und dann mit den weiteren Reaktionspartnern versetzt.

Die Umsetzung von olefinisch ungesättigter Verbindung mit Kohlenmonoxid und Wasserstoff erfolgt im allgemeinen bei Drücken von etwa 0,1 bis etw 300 MPa, vorzugsweise 1 bis 15 MPa, wobei Kobaltkatalysatoren im allgemeinen höhere Drücke erfordern als Rhodiumkatalysatoren. Die Zusammensetzung des Synthesegases, d.h. das Volumenverhältnis von Kohlenmonoxid und Wasserstoff kann sich über weite Bereiche erstrecken und z.B. zwischen 1:10 bis 10:1 variiert werden. Im allgemeinen setzt man Gasgemische ein, in denen das Volumenverhältnis von Kohlenmonoxid zu Wasserstoff etwa 1:1 ist oder von diesem Wert in der einen oder anderen Richtung nur wenig abweicht.

Die Reaktionstemperatur liegt zwischen etwa 20 und 180°C, bevorzugt werden 80 bis 150°C. Kobaltkatalysatoren erfordern im allgemeinen höhere Temperaturen als Katalysatoren auf Basis von Rhodium.

Die Umsetzung der in flüssiger oder gasförmiger Phase vorliegenden Reaktionspartner erfolgt in konventionellen Reaktoren. Der Ablauf der Umsetzung wird maßgeblich dadurch beeinflußt, daß im Fall der homogenen Systeme eine flüssige und eine gasförmige Phase, im Fall der heterogenen Systeme zwei flüssige und eine gasförmige Phase miteinander in innige Berührung gelangen. Es ist daher erforderlich, eine möglichst große Berührungsfläche zwischen den Phasen zu erzeugen. Daher führt man Synthesegas und gegebenenfalls auch Olefin bevorzugt über Verteilungsvorrichtungen der flüssigen Phase zu. Im Fall der mit heterogener Katalysatorphase durchgeführten Hydroformylierungsreaktion empfiehlt es sich, das Reaktionsgemisch intensiv zu rühren. Gegebenenfalls kann der wäßrigen Phase auch ein Lösungsvermittler zugesetzt werden, um die Löslichkeit der olefinischen Verbindung im Katalysator zu verbessern. Die Umsetzung kann absatzweise oder, bevorzugt, kontinuierlich durchgeführt werden.

Das erfindungsgemäße Verfahren ist mit Erfolg auf die Umsetzung von Monoolefinen, Polyolefinen, cyclischen Olefinen und Derivaten dieser ungesättigten Verbindungen anwendbar. Hinsichtlich der Molekülgröße unterliegen die eingesetzten Olefine keiner Beschränkung, die Arbeitsweise bewährt sich bei Verbindungen mit 2 bis 40 Kohlenstoffatomen. Die olefinisch ungesättigten Verbindungen können geradkettig oder verzweigt, die Doppelbindungen end- oder innenständig sein. Beispiele für Olefine, die in dem neuen Prozeß verwendet werden können, sind Ethylen, Propylen, Butyn-1, Buten-2, Penten-1, 2-Methylbuten-1, Hexen-1, Hexen-2, Hepten-1, Octen-1, Octen-3, 3-Ethylhexen-1, Decen-1, Undecen-3, 4,4-Dimethylnonen-1, Dicyclopentadien, Vinylcyclohexen, Cyclooctadien, Styrol, 2-Vinylnaphthalin. Derivate der genannten Olefinarten, die nach der beanspruchten Arbeitsweise hydroformyliert werden können, sind z.B. Alkohole, Aldehyde, Carbonsäuren, Ester, Nitrile und Halogenverbindungen, Allylalkohol, Acrolein, Methacrolein, Crotonaldehyd, Methylacrylat, Ethylcrotonat, Diethylfumarat, Diethylmaleinat, Acrylnitril. Mit besonderem Erfolg wird das Verfahren zur Hydroformylierung von Olefinen und Olefinderivaten mit 2 bis 20 Kohlenstoffatomen eingesetzt.

Ist in einer der als Katalysator eingesetzten Komplexverbindung einer der Carbon-Liganden chiral, d.h. besitzt er kein Symmetrieelement zweiter Art nach Schönflies, so treten in den Hydroformylierungsprodukten optische Induktionen auf, sofern die Einsatzstoffe prochiral sind und der Metallkomplex-Katalysator in optisch reiner Form verwendet wird. Auf diese Weise lassen sich chirale Produkte herstellen.

Besonders für die Anwendung in der Hydroformylierung eignet sich beispielsweise die folgende Carbenvorstufe der Formel (II):

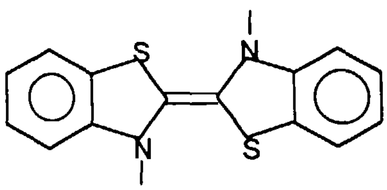

b) Verwendung von Verbindungen der Formel (I) in der Heck-Reaktion

Aromatische Olefine, insbesondere Zimtsäurederivate, Styrole und Stilbene, haben technische Bedeutung beispielsweise als Feinchemikalien, als Ausgangsstoffe für Polymere, als UV-Absorber und als Vorprodukte für pharmazeutische Wirkstoffe. Eine häufig angewandte Methode zu ihrer Synthese ist die Heck-Reaktion, d.h. die Umsetzung von Iod- oder Bromaromaten und in Ausnahmefällen Chloraromaten mit Olefinen in Gegenwart von Palladiumkatalysatoren. Übersichten, die diese Methodik ausführlich beschreiben, findet man beispielsweise in R.F. Heck, Acc, Chem. Res. 1979, 12, 146; R.F. Heck, Org. React. 1982, 27, 345; R.F. Heck, Palladium Reagents in Synthesis, Academic Press, London 1985.

Die als Einsatzstoffe verwendeten aromatischen Halogenverbindungen entsprechen vorzugsweise der allgemeinen Formel (III):

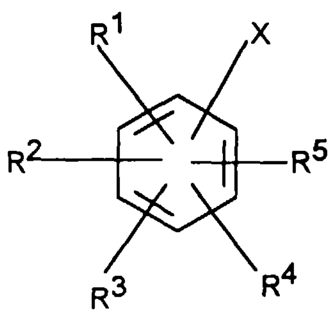

(III)

In dieser Formel bedeuten X Fluor, Chlor, Brom, Jod, $R^1$ bis $R^5$ unabhängig voneinander Wasserstoff, Alkylreste mit 1 bis 8 Kohlenstoffatomen, Alkoxyreste mit 1 bis 8 Kohlenstoffatomen, Acyloxyreste mit 1 bis 8 Kohlenstoffatomen, $-C_6H_5$, $OC_6H_5$, Fluor, Chlor, Brom, Jod, -OH, $-NO_2$, $-S(O)O_2CF_3$, -CN, -COOH, -CHO, $-SO_3H$, $-SO_2$ ($C_1$- bis $C_8$-alkyl), -SO($C_1$- bis $C_8$-alkyl), $-NH_2$, -NH($C_1$- bis $C_8$-alkyl), -N($C_1$- bis $C_8$-alkyl)$_2$, -C(hal)$_3$ (hal = Halogen), -NHCO($C_1$- bis $C_4$-alkyl), -COO($C_1$- bis $C_8$-alkyl), $-CONH_2$, -CO($C_1$- bis $C_8$-alkyl), -NHCOOH, -NCOO($C_1$- bis $C_4$-alkyl), $-COC_6H_5$, -$COOC_6H_5$-$PO(C_6H_5)_2$ und -PO($C_1$- bis $C_4$-alkyl)$_2$. Insbesondere sind $R^1$ bis $R^5$ unabhängig voneinander Wasserstoff, Alkylreste mit 1 bis 8 Kohlenstoffatomen, Alkoxyreste mit 1 bis 8 Kohlenstoffatomen, $-C_6H_5$, Fluor, Chlor, $-NO_2$, -CN, -COOH, -COO($C_1$- bis $C_8$-alkyl), $-CONH_2$, -CO($C_1$-bis $C_8$-alkyl), $-COC_6H_5$ und $-PO(C_6H_5)_2$. Einer der Reste $R^1$ bis $R^5$ kann auch der Gruppierung

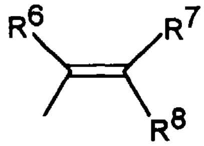

entsprechen, in der $R^6$ für Wasserstoff, Alkylreste mit 1 bis 8 Kohlenstoffatomen, Alkoxyreste mit 1 bis 8 Kohlenstoffatomen, den Phenylrest oder Fluor steht und $R^7$ und $R^8$ unabhängig voneinander Wasserstoff, -CN, -COOH, -COO($C_1$- bis $C_8$-alkyl), $-CONH_2$, -CONH($C_1$- bis $C_4$-alkyl), -CON($C_1$-$C_4$-alkyl)$_2$, Fluor, $-COOC_6H_5$, ($C_1$- bis $C_8$-alkyl)$C_6H_4$, -$PO(C_6H_5)_2$, -PO[($C_1$-bis $c_4$-alkyl)]$_2$, $-COC_6H_5$, -CO($C_1$- bis $C_4$-alkyl), Alkoxyreste mit 1 bis 4 Kohlenstoffatomen, -

$NH(C_1$- bis $C_4$-alkyl), $-PO_3H$, $-SO_3H$, $-SO_3(C_1$- bis $C_4$-alkyl), $-SO_2(C_1$- bis $C_4$-alkyl) oder $-OC_6H_5$ sind.

Reaktionspartner der vorstehend beschriebenen aromatischen Halogenverbindung sind Olefine der allgemeinen Formel (IV):

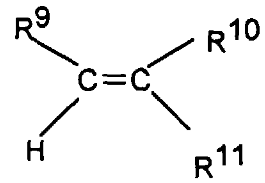

(IV)

Die Reste $R^9$, $R^{10}$ und $R^{11}$ haben die oben wiedergegebene Bedeutungen. In der Formel (III) steht X bevorzugt für Chlor und Brom. Ferner sind $R^1$, $R^2$, $R^4$ und $R^5$ insbesondere gleich und bedeuten Wasserstoff. $R^3$ ist insbesondere der Methylrest, der Methoxyrest, $-NO_2$ und -C(O)H. In Formel (IV) bedeutet $R^9$ vorzugsweise Wasserstoff, einem Alkylrest mit 1 bis 8 Kohlenstoffatomen und insbesondere Wasserstoff. $R^{10}$ und $R^{11}$ sind unabhängig voneinander bevorzugt Wasserstoff, -CN, -COOH, $-COO(C_1$- bis $C_8$-alkyl), $-COOC_6H_5$, $-COC_6H_5$, $-CO(C_1$-bis $C_4$-alkyl) und besonders bevorzugt -CN, -COOH, $-COO(C_1$- bis $C_8$-alkyl) und $-COOC_6H_5$. Insbesondere sind $R^9$ und $R^{11}$ gleich und stehen für Wasserstoff.

Die Reaktion der Ausgangsstoffe erfolgt bei Temperaturen von 20 bis 200°C. In vielen Fällen hat es sich bewährt, bei 60 bis 180°C, vorzugsweise 100 bis 160°C zu arbeiten.

Im allgemeinen setzt man die Reaktanten in einem inerten, organischen Lösungsmittel um. Gut geeignet sind dipolar aprotische Lösungsmittel, wie Dialkylsulfoxide, N,N-Dialkylamide aliphatischer Carbonsäuren oder alkylierte Lactame. Bevorzugt verwendet werden Dimethylsulfoxid, Dimethylacetamid, Dimethylformamid, N-Methylpyrrolidon, Amine und Polyether.

Im Verlauf der Reaktion wird Halogenwasserstoff abgespalten, den man zweckmäßig durch Zusatz einer Base abfängt. Als Base geeignet sind primäre, sekundäre oder tertiäre Amine, z.B. Alkylamine, Dialkylamine, Trialkylamine, die alicyclisch oder offenkettig sein können, sowie Alkali- oder Erdalkalisalze der Kohlensäure oder aliphatischer oder aromatischer Carbonsäuren, z.B. die Carbonate, Hydrogencarbonate oder Acetate der Metalle Lithium, Natrium, Kalium, Calzium und Magnesium.

Aufgrund ihrer hohen Aktivität und Stabilität reichen bereits kleine Mengen der neuen Katalysatoren zur Durchführung der Umsetzung aus. Das Verfahren ist daher sehr wirtschaftlich und ökologisch vorteilhaft, weil Abfallprodukte vermieden werden und energieaufwendige Aufarbeitungsverfahren entbehrlich sind. Üblicherweise setzt man die Katalysatoren, bezogen auf die aromatische Halogenverbindung, in Mengen zwischen $10^{-4}$ bis 5 mol-%, vorzugsweise $10^{-2}$ bis 0,5 mol-% ein.

Die Katalysatoren werden vorzugsweise im Reaktionsgemisch aus gängigen Palladiumverbindungen erzeugt, besonders bevorzugt ist die Erzeugung in situ aus einer Palladiumverbindung und einem der oben beschriebenen Alkene, die als Carbenvorstufen dienen.

Der Katalysator kann jedoch auch vor der eigentlichen Reaktion isoliert und synthetisiert werden.

Die Reihenfolge der Zugabe der Reaktionspartner ist im allgemeinen unkritisch.

Als Palladiumvorstufen eignen sich insbesondere die Palladium(II)-halogenide, Palladium(II)-acetat, Palladium(II)-acetylacetonat, Nitrilkomplexe der Palladium(II)-halogenide, Bis(dibenzylidenaceton)palladium und Bis(1,5-cyclooctadien)palladium(0).

Die Herstellung der Katalysatoren in einem eigenen Reaktionsschritt erfolgt aus einfachen Verbindungen, d.h. Palladiumsalzen oder aus Komplexverbindungen des Palladiums durch Ligandenaustausch durch Anlagerungs-, Eliminierungs- und/oder Substitutionsreaktionen. Die Carbene werden entsprechend ihrer Beständigkeit entweder in freier Form als Lösung eingesetzt oder, bevorzugt, im Reaktionsgemisch aus Verbindungen hergestellt, die unter den Reaktionsbedingungen in Carbene überführt werden können. Eine bevorzugte Erzeugungsmethode ist die oben beschriebene Verwendung geeigneter Alkene, die unter den Reaktionsbedingungen mit einer Palladiumverbindung einen Carbenkomplex der Formel (I) ausbilden, ebenso möglich ist die Deprotonierung von entsprechenden Salzen, gegebenenfalls durch Zusatz von Basen, wie Metallalkoholate, Metallhydride, Halogenmetallate oder Metallamide.

Die Aktivität der Katalysatoren läßt sich durch Zusatz von Alkalisalzen, Erdalkalisalzen oder Salzen von Übergangsmetallen der 6. bis 8. Nebengruppe erhöhen. Insbesondere der Zusatz von Halogeniden und Pseudohalogeniden wie Cyanid, bewirkt bei der Umsetzung von Chloraromaten erhebliche Ausbeutesteigerung und erhöht die Lebensdauer des homogenen Katalysators. Das gleiche Ergebnis erzielt man auch bei Zusatz von Tri- und Tetraalkylammonium-Salzen sowie den entsprechenden Phosphonium- und Arsoniumsalzen.

Besonders geeignet für die Anwendung in der Heck-Reaktion ist beispielsweise die folgende Carbenvorstufe der

Formel (II):

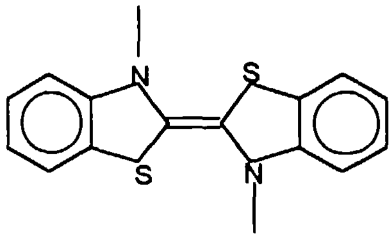

Die Erfindung wird durch die Beispiele näher erläutert, ohne sie dadurch beschränken zu wollen.

Beispiele

Beispiel 1 N-Methyl-benzthiazoliumiodid

45,0 g (333 mmol) Benzthiazol (frisch destilliert) werden in 25 ml Dimethylformamid gelöst und bei 23°C mit 47,3 g (333 mmol) Methyliodid versetzt. Nach 2 Stunden bei 75°C wird das Rohprodukt abfiltriert, mit Diethylether nachgewaschen und aus einer siedenden Mischung aus Ethanol/Wasser (5:1) umkristallisiert. Man erhält 74,3 g N-Methyl-benzthiazoliumiodid (81 % der Theorie) mit Fp. 209°C.

Beispiel 2 Bis-[N-methyl-benzthiazolinyliden-(2)]

(Carbenvorstufe der Formel II)

Zu einer Suspension von 16,0 g (58 mmol) N-Methyl-benzthiazoliumiodid in 28 ml Dimethylformamid werden bei 23°C 9 ml (58 mmol) Triethylamin innerhalb von 20 Minuten zugetropft, wonach sich eine klare braune Lösung gebildet hat. Nach 1 Stunde Rühren bei 0°C (Eiskühlung) kristallisiert das Produkt aus. Nach Absaugen, Nachwaschen mit kaltem Aceton und Trocknen im Ölpumpenvakuum erhält man 5,2 g Produkt (60 % der Theorie) mit Fp. 140°C. Massenspektrum (EI, 70eV): m/z 298 (45 %, $M^+ - CH_3$).

Beispiel 3 Heck Reaktion

15 mmol 4-Bromacetophenon und 22 mmol Acrylsäure-2-ethylhexylester wurden in 30 ml Dimethylacetamid gelöst. Unter Rühren wurden eine Spatelspitze 2,6-Di-t-butylphenol, 18 mmol Natriumacetat, 0,1 mol-% $Pd(DBA)_2$ (DBA = Dibenzylidenaceton), bezogen auf 4-Bromacetophenon und 1,1 Äquivalente Bis-[N-methyl-benzthiazolinyliden-(2)], bezogen auf Palladium, zugegeben. Es wurde 6 h bei 135°C gerührt.
Ausbeute (gaschromatographisch bestimmt):
73 % 4-Acetylzimtsäure-2-ethylhexylester
25 % 4-Bromacetophenon

Beispiel 4 Heck Reaktion

15 mmol 4-Bromacetophenon und 22 mmol Acrylsäure-2-ethylhexylester wurden in 30 ml Dimethylacetamid gelöst. Unter Rühren wurden eine Spatelspitze 2,6-Di-t-butylphenol, 18 mmol Natriumacetat, 0,1 mol-% Palladium(II)-acetat, bezogen auf 4-Bromacetophenon und 1,1 Äquivalente Bis-[N-methyl-benzthiazolinyliden-(2)], bezogen auf Palladium, zugegeben. Es wurde 6 h bei 135°C gerührt.
Ausbeute (gaschromatographisch bestimmt):
100 % 4-Acetylzimtsäure-2-ethylhexylester

Beispiel 5 Hydroformylierung von Octen

59 mg Bis-[N-methyl-benzthiazolinyliden-(2)] (MW = 252,36, 0,233 mmol) und 60 mg Rhodium(III)-acetat (0,233 mmol) wurden in 60 ml abs. Toluol gelöst und in einen 200 ml Stahlautoklav (Fa. NOVA) gegeben. Der Autoklav wurde verschlossen, 30 bar Synthesegas (CO : $H_2$ = 1:1) aufgedrückt und der Ansatz unter kräftigem magnetischen

Rühren auf 120°C erwärmt (Präformierung). Nach 3 Stunden Reaktionszeit wurden 38 ml (240 mmol) 1-Octen zudosiert, und es wurde bei 120°C und in einem Druckbereich von 30 bis 40 bar Synthesegasdruck hydroformyliert. Nach 3 Stunden wurde der Autoklav entspannt und auf Raumtemperatur abgekühlt. Gemäß Gaschromatographie wurde ein Umsatz von 74 % in Bezug auf 1-Octen erzielt, das Verhältnis von 1-Nonanal zu 2-Methyloctanal betrug 35:65.

## Patentansprüche

**1.** Katalysator für Kohlenstoff-Kohlenstoff-Verknüpfungsreaktionen, enthaltend eine Komplexverbindung der Formel (I)

$$[L_a M_b X_c]^{n+}(A)_n \qquad (I)$$

worin die Symbole und Indizes folgende Bedeutungen haben:

M ist gleich oder verschieden Pd, Rh, Ni, Ru, Co;
X bedeutet gleich oder verschieden an das Zentralatom gebundene ein- oder mehrzähnige, geladene oder ungeladene Liganden;
A ist ein einfach geladenes Anion oder das chemische Äquivalent eines mehrfach geladenen Anions,
b ist eine natürliche Zahl von 1 bis 3;
a ist eine natürliche Zahl von 1 bis 5 • b;
c ist Null oder eine natürliche Zahl von 1 bis 4 • b;
n ist Null oder eine natürliche Zahl von 1 bis 6, mit der Maßgabe, daß $c + n > 0$ ist, und
L ist gleich oder verschieden ein cyclisches Monocarben mit 5, 6 oder 7 Ringgliedern mit mindestens einem Stickstoffatom in Nachbarschaft zu dem Carbenkohlenstoff oder ein Dicarben, bei dem zwei solcher cyclischen Monocarbene über eine Brücke verbunden sind, mit Ausnahme solcher Verbindungen, in denen das cyclische Carben ein Imidazol- oder Pyrazolsystem ist.

**2.** Katalysator nach Anspruch 1, dadurch gekennzeichnet, daß das cyclische Mono- oder Dicarben eines der folgenden 5-, 6- oder 7-gliedrigen Ringsysteme aufweist

a) Fünfringsysteme aus der Gruppe:

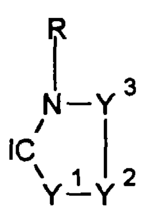 , 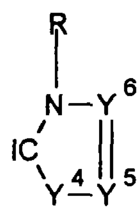 , 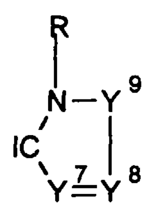

wobei

$Y^1, Y^2, Y^3, Y^4, Y^9$ -NR-, -S-, $-CH_2-$, -CHR'-, -CR'R'- und
$Y^5, Y^6, Y^7, Y^8$ gleich oder verschieden = N-, -CH =, = CR' bedeuten,

b) Sechsringsysteme aus der Gruppe:

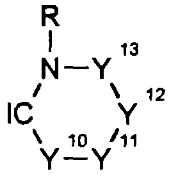 , 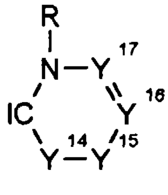 , 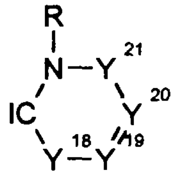 , 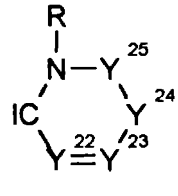 , 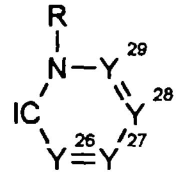

wobei

$Y^{10, 11, 12, 13, 14, 15, 18, 21, 24, 25}$ gleich oder verschieden -NR-, -S-, $-CH_2-$, -CHR'-, -CR'R'- und

$Y^{16, 17, 19, 20, 22, 23, 26, 27, 28, 29}$ gleich oder verschieden = N-, = CH-, = CR'- bedeuten;

c) Siebenringsysteme aus der Gruppe:

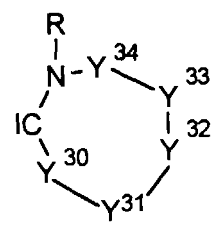 , 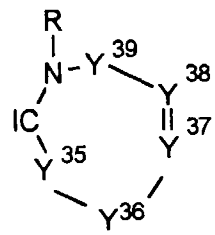 , 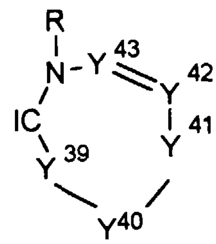 , 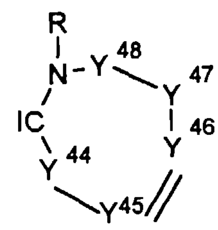 ,

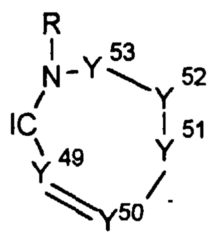 , 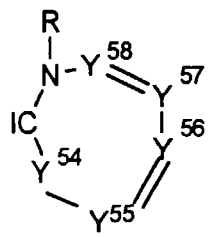 , 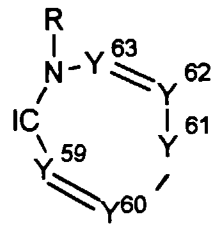 , 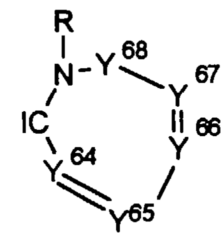

wobei

$Y^{30-34, 35, 36, 38-41, 44, 47, 48, 51-54, 61, 68}$ gleich oder verschieden -NR-, -S-, $-CH_2-$, -CHR'-, -CR'R'-,

$Y^{32, 33, 41, 47, 51, 52, 61}$ auch -O- und

$Y^{37, 38, 42, 43, 45, 46, 49, 50, 55-60, 62-67}$ gleich oder verschieden = N-, -CH =, -CR' = bedeuten, mit den Maßgaben, daß Schwefel- und/oder Sauerstoffatome nicht benachbart sein dürfen und daß ein Ring nicht mehr als vier Stickstoffatome enthalten darf;

R,R' sind gleich oder verschieden H, geradkettige oder verzweigte, gegebenenfalls sulfonierte Alkylreste mit vorzugsweise 1 bis 7 Kohlenstoffatomen, gegebenenfalls sulfonierte aliphatische mono- oder polycyclische Reste mit vorzugsweise 5 bis 18 Kohlenstoffatomen, gegebenenfalls sulfonierte Alkenylgruppen mit vorzugsweise 2 bis 5 Kohlenstoffatomen, gegebenenfalls sulfonierte Arylgruppen mit vorzugsweise 6 bis 14 Kohlenstoffatomen oder gegebenenfalls sulfonierte Arylalkylreste mit vorzugsweise 7 bis 19 Kohlenstoffatomen bedeuten,

R' kann auch F, Cl oder eine geradkettige oder verzweigte Alkoxygruppe mit 1 bis 7 Kohlenstoffatomen sein, zwei benachbarte Reste R und/oder R' können gemeinsam jeweils auch gleiche oder verschiedene anellierte und gegebenenfalls sulfonierte Reste mit 3 bis 7 Kohlenstoffatomen bedeuten.

**3.** Katalysator nach Anspruch 1 oder 2, gekennzeichnet durch ein cyclisches Mono- oder Dicarben, enthaltend ein 5-, 6- oder 7-Ringsystem aus der Gruppe:

a) 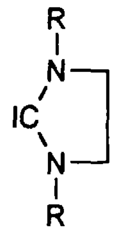 , 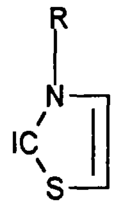 , 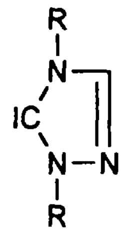 , 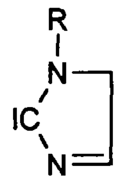 ,

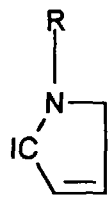 , 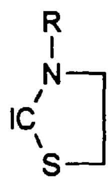 , 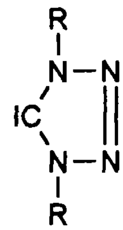 , 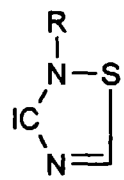

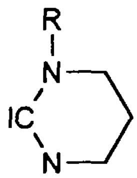 , 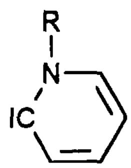 , 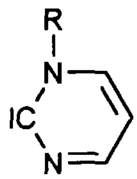 , 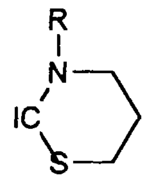 , 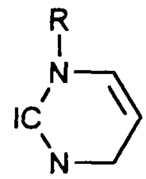

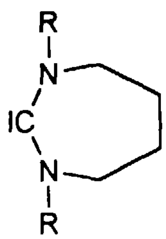 , 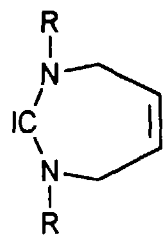 , 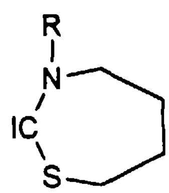 , 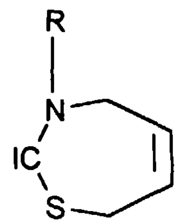 ,

wobei ein bis vier Wasserstoffatome des Ringsystems gegebenenfalls durch gleiche oder verschiedene Gruppen R'ersetzt sein können und wobei R, R' gleich oder verschieden die gleichen Bedeutungen wie in Anspruch 2 haben.

**4.** Verwendung einer Komplexverbindung der Formel (I) nach einem oder mehreren der Ansprüche 1 bis 3 als Katalysator für Kohlenstoff-Kohlenstoff-Verknüpfungsreaktionen.

**5.** Verwendung nach Anspruch 4, dadurch gekennzeichnet, daß die Kohlenstoff-Kohlenstoff-Verknüpfungsreaktion eine Heck-Reaktion ist.

**6.** Verwendung nach Anspruch 4, dadurch gekennzeichnet, daß die Kohlenstoff-Kohlenstoff-Verknüpfungsreaktion eine Hydroformylierung ist.

**7.** Verfahren zur Knüpfung von Kohlenstoff-Kohlenstoff-Bindungen, dadurch gekennzeichnet, daß das oder die zu verknüpfenden Edukte in Gegenwart einer Komplexverbindung der Formel (I) nach einem oder mehreren der Ansprüche 1 bis 3 umsetzt.

**8.** Verfahren nach Anspruch 7, dadurch gekennzeichnet, daß die Komplexverbindung der Formel (I) in der Reaktionsmischung in situ aus einer Pd-, Rh-, Ni-, Ru- oder Co-Verbindung und einem Alken der Formel (II) gebildet wird,

$$Z = Z \qquad \text{(II)}$$

in der Z ein Ringsystem mit 5, 6 oder 7 Ringgliedern ist, wobei das Kohlenstoffatom, an dem sich die exocyclische Doppelbindung befindet, direkt an ein Stickstoff und ein weiteres Heteroatom gebunden ist und wobei die beiden Gruppen Z auch über eine Brücke verbunden sein können; mit der Ausnahme von Verbindungen, in denen Z ein Imidazol- oder Pyrazolsystem ist.

**9.** Verfahren nach Anspruch 7 und/oder 8, dadurch gekennzeichnet, daß eine olefinisch ungesättigte Verbindung mit Kohlenmonoxid und Wasserstoff bei Drücken von 0,1 bis 300 MPa umgesetzt wird, wobei M in der Formel (I) für Co oder Rh steht.

**10.** Verfahren nach Anspruch 7 und/oder 8, dadurch gekennzeichnet, daß ein Halogenaromat mit einem Olefin, das mindestens ein Wasserstoffatom an der Doppelbindung enthält, umgesetzt wird, wobei M in der Formel (I) für Pd steht.

Europäisches Patentamt

# EUROPÄISCHER RECHERCHENBERICHT

Nummer der Anmeldung
EP 97 20 0759

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | ANGEWAND CHEMIE, INTERNATIONAL EDITION ENGLISH, Bd. 34, Nr. 21, 1995, Seiten 2371-2374, XP002001061 HERRMANN, W.A. ET AL.: "Metal complexes of n-heterocyclic carbenes" | 1-7,9,10 | B01J31/22 B01J31/18 C07C67/343 |
| A | *das ganze dokument* | 8 | |
| | ----- | | |
| | | | RECHERCHIERTE SACHGEBIETE (Int.Cl.6) |
| | | | B01J C07C |

Der vorliegende Recherchenbericht wurde für alle Patentansprüche erstellt

| Recherchenort | Abschlußdatum der Recherche | Prüfer |
|---|---|---|
| DEN HAAG | 3.Juli 1997 | Faria, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)